# EUROPEAN PATENT APPLICATION

(11) **EP 1 607 077 A1**
(43) Date of publication of application: **21.12.2005**
(21) Application number: 05405378.0
(22) Date of filing: 13.06.2005
(51) Int. Cl.: A61F 9/007

(54) **Phacoemulsification needle**

(30) Priority: 14.06.2004 US 866187
(71) Applicant: Akahoshi, Takayuki, Tokyo 104-0051 (JP); Nallakrishnan, Ravi, Westmont, IL 60559 (US)
(72) Inventor: Akahoshi, Takayuki, Tokyo 104-0051 (JP)
(74) Representative: Liebetanz, Michael

(57) **Abstract**

A phacoemulsification needle according to the invention comprises a shaft, a tip disposed at a distal end of said shaft and an aspiration lumen extending through said shaft and said tip. The tip has an opening communicating with said aspiration lumen. The shaft comprises at least a portion which has a ribbed outer surface. This needle minimizes heat generation and can therefore be used with or without sleeve.

## Description

### Field of the invention

This invention relates to a phacoemulsification needle for an ultrasonic surgical instrument, the needle being designed for promoting cavitation in eye tissue and for the removal of fragmented lens from the eye.

### Background of the invention

Phacoemulsification has become the preferred form of cataract, i.e. a cloudy eye lens, removal. One of the main advantages of phacoemulsification is, that only a small incision into the cornea or sclera of an eye is needed to remove the cataract. Furthermore, the removal of the cataract can be done very quickly. After the cataract is removed, an intraocular lens is inserted to replace the original lens.

The phacoemulsification technique uses a hand held microsurgical tool known as phacoemulsifier. This phacoemulsifier comprises a handpiece and a small diameter needle with a tip to be inserted into the small incision of the eye. The needle and therefore the tip are vibrated by an ultrasonic source. It breaks the cataract into small fragments and pieces, which are sucked out through the same tip in a controlled manner. The tip is therefore designed for emulsifying, fragmenting and/or cutting tissue and also comprises a central hollow bore or lumen for the suction or aspiration of the fragments.

During the procedure, an irrigation solution is introduced to maintain the pressure and to prevent the eye from collapsing. In order to introduce the irrigation solution, the needle is usually covered by a sleeve and the solution flows via the space between this sleeve and the needle. The solution is therefore also used to cool the tip, which is heating up during the phacoemulsification.

Heat generated by the needle or tip degenerates the collagen of the cornea or sclera in the wound. If the collagen is degenerated by the heat, the wound will be damaged and cannot be self-sealed at the end of the surgery. An unsealed wound can cause anterior chamber collapse, which will result in severe corneal endothelial cell damage and most serious intraocular infection. Suturing the heat damaged tissue may attain sealing of the wound, however, it will cause deformity of the cornea and result in the large post-operative astigmatism. Thus decreasing the risk of the thermal damage of the wound by the tip is one of the most important points of cataract surgery by phacoemulsification.

US-A-5'653'724 discloses a phacoemulsification needle which is angled to provide a more comfortable ergonomic angle during phacoelmulsification and lens cortex removal. This angled needle is also considered to produce less heat when emulsifying the lens. Another angled phacoemulsification needle with a concentric sleeve is disclosed in US-5'993'409.

Different shapes of phacoemulsification needles with slits, a second infusion hole and/or with increased outside diameter at the distal end of the needle body and the needles being surrounded by sleeves are described in US-A-5'989'209, US-A-6'159'175, EP-A-1'103'238, WO 00/74615 and US 2002/0099325. US-A-5'989'209 furthermore discloses the use of a ribbed insert together with a sleeve. This shall permit continued irrigation fluid flow while reducing the contact area between the sleeve and the ribbed insert, thereby reducing the risk of thermal damage to the entry wound of the eye.

Other techniques for cataract removal use laser energy to remove the cataract. A laser/aspiration probe is used for breaking and removing the lens. A separate infusion or irrigation probe is used for the irrigation solution.

### Summary of the invention

It is an object of the invention to provide a phacoemulsification needle which minimizes the heat generation and which can therefore also be used without a sleeve.

This object is achieved with a phacoemulsification needle comprising
a shaft,
a tip, disposed at a distal end of said shaft and
an aspiration lumen extending through said shaft and said tip, the tip having an opening communicating with said aspiration lumen,
wherein said shaft comprises at least one portion having a ribbed outer surface.

The ribbed outer surface is preferably formed by spiral ridges or a helix. Preferably, the tip has an outer diameter being at least approximately the same as an outer diameter of said ribbed outer surface of said shaft. In an advantageous embodiment, the needle comprises a tapered portion connecting said ribbed outer surface and said tip. The ribbed outer surface preferably extends at least approximately along the whole length of said shaft. The tip may be angled. In an advantageous embodiment, the opening has a larger diameter than the part of the aspiration lumen extending within said shaft. The aspiration lumen may comprise a conical step within the tip. This conical step has preferably an angle of approximately 30°.

The object is further achieved with a phacoemulsification needle comprising
a shaft with a first and a second end,
a tip, disposed at the first end of said shaft and
an aspiration lumen extending through said shaft and said tip, the tip having an opening communicating with said aspiration lumen,
wherein said shaft comprises at least one portion having protrusions on its outer surface.

The same object is achieved with a phacoemulsification needle comprising
a shaft with a first end and a second end,
a tip, disposed at the first end of said shaft and
an aspiration lumen extending through said shaft and said tip, the tip having an opening communicating with said aspiration lumen,
wherein said shaft comprises at least one portion having depressions in its outer surface.

Since the shaft comprises depressions or protrusions, such as ribs or grooves, the surface of the tip is increased and the cooling effect of the tip is markedly enhanced.

In a preferred embodiment, the ribs or grooves have a spiral or helical form, or they are rings, or they are rectilinear or they have a concave or convex shape.

If the ribs or grooves have a helical form, it is preferred that the helix angle (pitch angle) is between 5 and 30 degrees. This ensures an optimum reflux of anterior chamber fluid and/or irrigation fluid along the tip in the direction from first (tip) end of the shaft towards the second end when the tip is used without an irrigation sleeve.

The needle according to the invention can be used with or without an irrigation sleeve.

In a preferred embodiment, the tip comprises a slit extending in the longitudinal direction of the needle and communicating with the aspiration lumen.. When a high vacuum setting is used with the phacoemulsifier, the anterior chamber, formed of the part of the lumen extending in the tip, becomes unstable when occlusion break occurs. In this case, small amount of irrigation fluid will continue to flow through the aspiration lumen. Furthermore, when occlusion surge occurs, the amount of surge can be reduced.

Further preferred embodiments of the invention are described in the dependent claims.

### Brief description of the drawings

The present invention will be more clearly understood with reference to the following detailed description of preferred embodiments, taken in conjunction with the accompanying drawings, in which
- Figure 1a: shows a perspective view of a needle according to the invention in a first embodiment,
- Figure 1b: the needle according to figure 1a in a side view,
- Figure 1c: in another side view,
- Figure 1d: shows a magnified part of the needle tip according to figure 1c;
- Figure 2a: shows a perspective view of a needle according to the invention in a second embodiment,
- Figure 2b: the needle according to figure 2a in a side view,
- Figure 2c: in another side view,
- Figure 2d: shows a magnified front view of the needle according to figure 2a;
- Figure 2e: shows a front view of the needle according to figure 2d;
- Figure 3a: shows a perspective view of a needle according to the invention in a third embodiment,
- Figure 3b: the needle according to figure 3a in a side view,
- Figure 3c: in another side view,
- Figure 3d: shows a magnified front view of the needle according to figure 3a and
- Figure 4a: shows a perspective view of a needle according to the invention in a fourth embodiment,
- Figure 4b: the needle according to figure 4a in a side view,
- Figure 4c: in another side view,
- Figure 4d: shows a magnified front view of the needle according to figure 4a;
- Figure 5: shows a perspective view of a needle according to the invention in a fifth embodiment;
- Figure 6: shows a perspective view of a needle according to the invention in a sixth embodiment;
- Figure 7: shows a perspective view of a needle according to the invention in a seventh embodiment;
- Figure 8: shows a perspective view of a needle according to the invention in an eighth embodiment;
- Figure 9a: shows a perspective view of a needle according to the invention in a ninth embodiment; and
- Figure 9b: shows side elevational view of the needle of Figure 9a.

### Description of the preferred embodiments

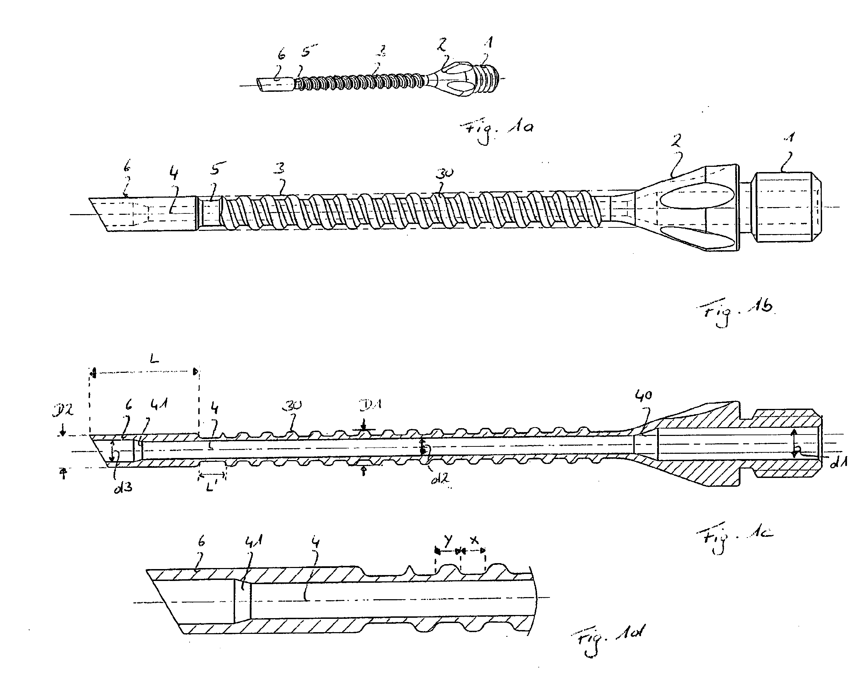

With reference to figures 1a and 1b, there is shown a phacoemulsification needle in accordance with the present invention. The needle may be formed from any conventional material as is known in the art for the manufacture of phacoemulsification needles. Usually, it is made of titan.

The needle comprises, starting with its proximal end and ending with its distal end (with reference to the surgeon), a threaded portion 1, a hub 2, a shaft 3 and a tip 6 having an opening. The threaded portion 1 and the hub 2 form a joint end enabling the needle to be fixed to a ultrasonic device of the phacoemulsification handpiece (not shown) in order to couple ultrasonic energy to the needle.

The needle further comprises, with reference to figures 1c and 1d, an aspiration lumen 4 extending through the whole longitudinal length of the needle shaft 3 and communicating with the opening of the tip for aspiration of irrigation fluid and therefore for removal of fragments and pieces of the cataract. Preferably, the lumen 4 comprises a first step 40 in the region of the hub 2 neighboring the shaft 3. The first diameter d1 of the part of the lumen extending within the hub 2 is therefore larger than the second diameter d2 of the lumen part extending within the shaft 2. This first step 40 has preferably a conical shape.

A second step 41, which has preferably also a conical shape, is located within the tip 6, wherein the second diameter d2 is smaller than a third diameter d3 of the lumen part extending within the shaft 2. The width of the opening of the tip is even larger than the third diameter d3.

With exception of the steps 40, 41, the lumen preferably has throughout its whole length the same size. In particular the lumen part extending in the tip 6 has a cylindrical shape.

In this embodiment, the shaft 3 is rectilinear. The shaft 3 comprises at least one portion which has a ribbed outer surface 30. These ribs extending radially from the outer side of the shaft are preferably built by spiral ridges or a helix. Other protrusions are however possible. This portion extends preferably at least approximately along the whole length of the shaft 3.

Disposed between this portion with the ribbed surface 30 and the tip 6 is a tapered portion 5, connecting these two. The tip 6, which is disposed at the distal end of the shaft 3, has preferably at least approximately the same outer diameter D2 as the outer diameter D1 of the shaft 3. The above mentioned second step 41 is preferably located in the tip 6 itself at a distance to the transition of the ball-shaped surface 60 to the surface of the shaft 3.

A preferred embodiment of the inventive needle has an outer diameter D1 of the shaft 2 and an outer diameter D2 of the tip of approximately 1.32 mm, an inner diameter D1' of the shaft 3 of approximately 0.956 mm, a first diameter d1 of the lumen 4 of approximately 1.32 mm, a second diameter d2 of approximately 0.72 mm, a third diameter d3 of approximately 0.9 mm, a length L of the tip 5 of approximately 4.385 mm and a distance x from the frontal end to the second step 41 of approximately 1.8 mm. The distance X between two ribs is approximately 0.5 mm, the thickness Y of a rib is also approximately 0.5 mm. The length L' of the tapered portion 5 is approximately 0.8 mm, wherein the transition areas between the tapered portion and the tip and the tapered portion and the shaft respectively comprise an angle of 30° each. The angle α of the first step 40 is approximately 34° and the angle β of the second step 41 approximately 30°. The total length of the needle is approximately 30 mm.

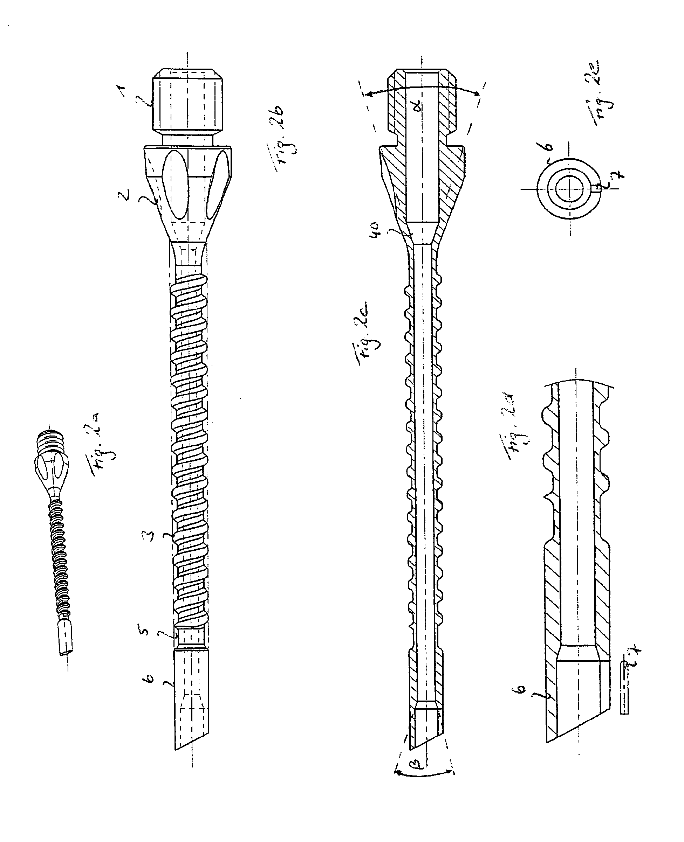

Figures 2a to 2d show a second preferred embodiment of the needle according to the invention. The needle is built in the same way as the needle according to the previously described figures. The only difference is, that this tip 6 comprises a slit 7 extending in longitudinal direction of the needle, which communicates with the aspiration lumen 4. This slit can be best seen in figures 2d and 2e.

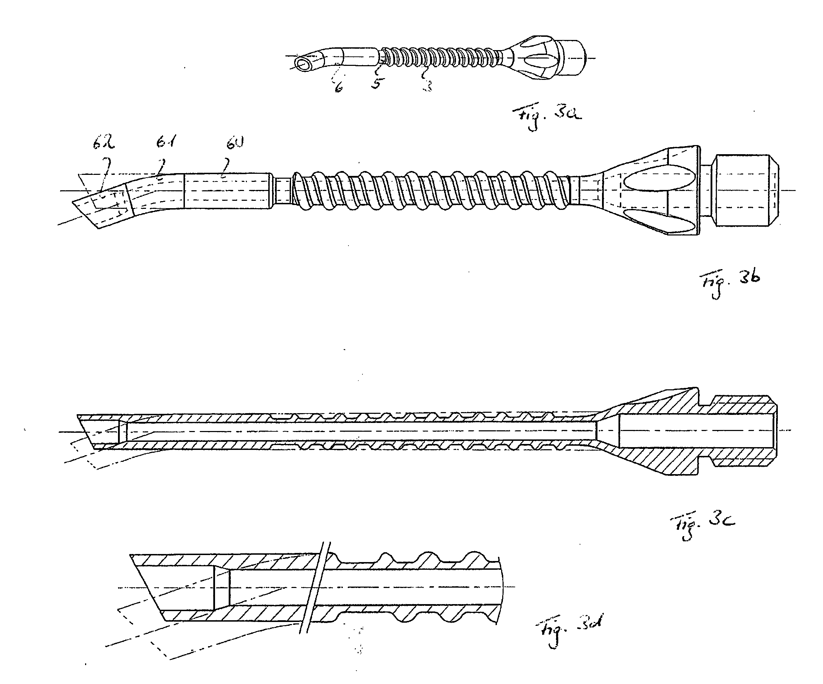

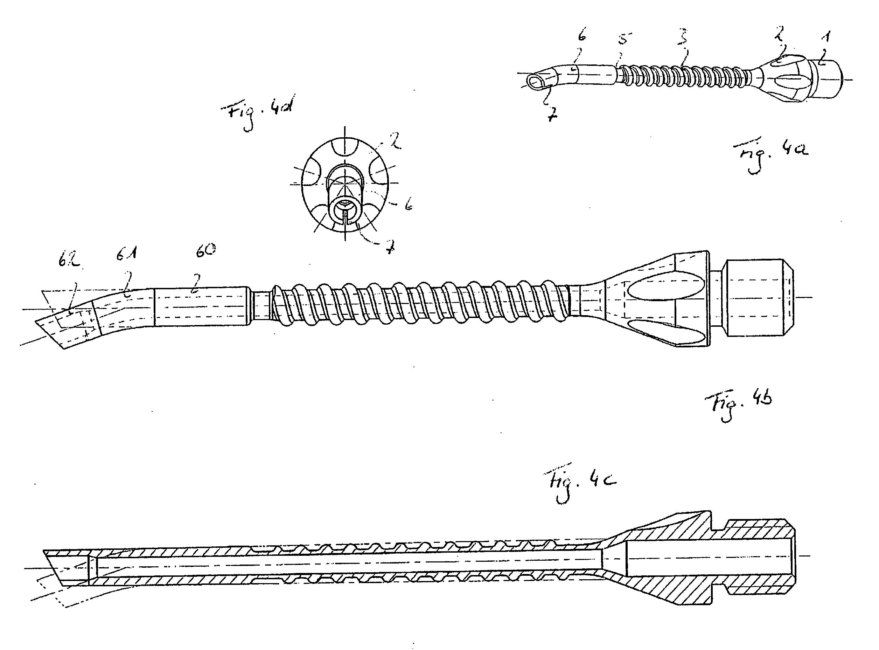

Figures 3a to 3d show a third preferred embodiment being similar to the embodiment according to figures 1a to 1d without the slit 7 and Figures 4a to 4d show a fourth preferred embodiment being similar to the embodiment according to figures 2a to 2d comprising the slit 7. The only difference to these figures is, that in the third and fourth embodiments the tip 6 is angled. The tip 6 consists of an extension portion 60 being disposed at the distal end of the tapered portion 5, followed by an angled portion 61 and an end portion 62. The end portion 62 has a flared end surface like the end portions of the first and second embodiments. This forms a large port or opening which can remove the lens material most efficiently, whereas the smaller diameter of the tip contributes to the stability of the anterior chamber formed by the lumen part within the tip. The angle between extension portion 60 and end portion 62 preferably lays between 17.5° and 20°.

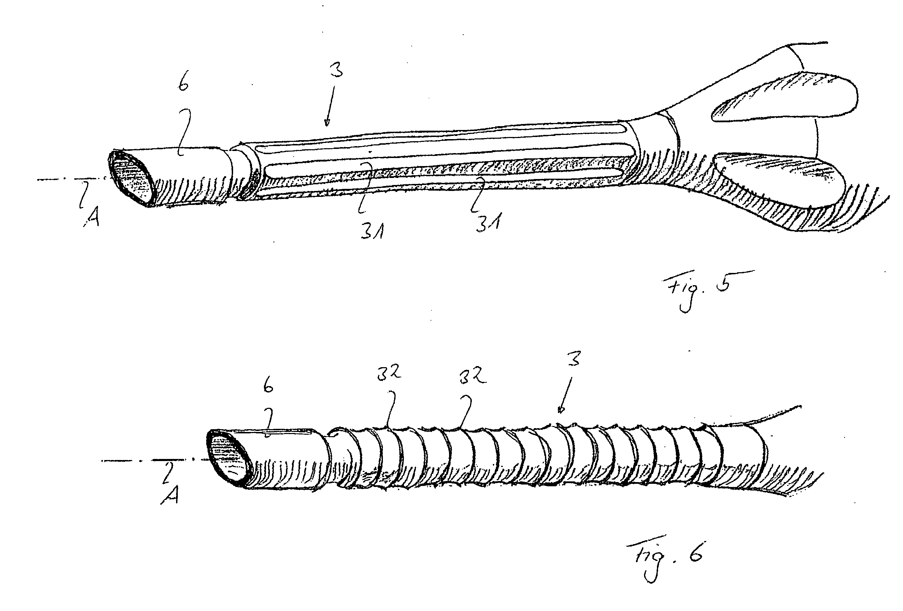

Figure 5 shows another preferred embodiment. In this embodiment, the shaft 3 comprises at least one portion which has one or more elongated grooves 31 extending at least approximately parallel to the longitudinal axis A of the needle shaft 3. In the embodiment shown the grooves 31 extend almost along the whole length of the shaft 3, they are rectilinear and they are arranged in an equidistant way from each other. However, these grooves 31 can also be arranged in a spiral (helical) way around the circumference of the needle shaft 3.

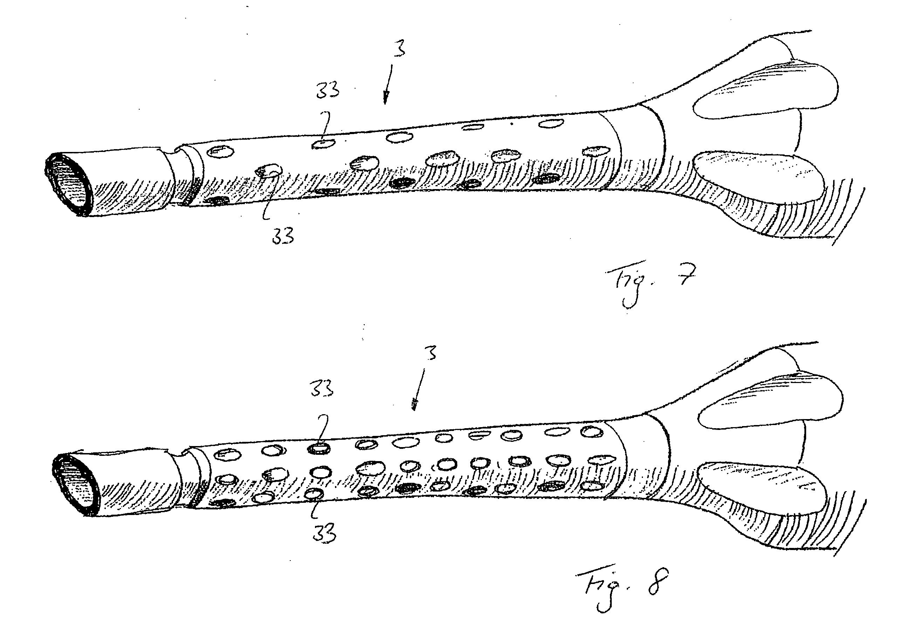

The embodiment according to Figure 6 comprises ribs 32 or grooves, each of them having the shape of a ring or an annulus extending around the outer surface of the needle shaft 3. In the embodiment shown, the ribs 32 or grooves describe a plane being perpendicular to the longitudinal axis A of the shaft 3. However, they can also be inclined in an angle. Preferably, the ribs 32 or grooves are arranged in an equidistant way. However, the spaces between the ribs 32 or grooves can vary. For example, it is possible that the distances become smaller or bigger the nearer the ribs 32 or grooves are located to the distal end.

Further embodiments are shown in Figures 7 and 8. In these embodiments, the outer surface of the shaft 3 comprises a multiple of punctiform depressions 33. These depressions 33 can all have the same size and depth or they can have different sizes and depths. Furthermore, it is possible that the sum of the depressed area amounts to almost half of the total surface area of the shaft or it can be far less than this amount.

The embodiments according to Figures 5 to 8 can comprise an angled tip 6 instead of the tip 6 extending along the longitudinal axis A.

Furthermore, the embodiments according to Figure 7 and 8 can also comprise instead of or in addition to the concave depressions convex protrusions in the same arrangement and/or size as the depressions shown in these figures.

It is furthermore possible to combine depressions and protrusions, for example grooves and ribs, on the same shaft.

In another preferred embodiment, the ribs or grooves can also be located on the inner wall of the lumen 4. These inner ribs or grooves can be an additional feature of a tip having the outer ribs, helix or grooves. However, the tip can also comprise ribs and grooves on the inner side only and the outer surface can be smooth.

The needle according to the invention minimizes heat generation and can therefore be used with or without sleeve. In all of these designs the surface area of the needle is increased by the various designs to reduce heat with or without sleeve. When the needles are used in addition with a sleeve the irrigation may be enhanced due to the shape of the outer surface of the needle. When a sleeve extends over a smooth surface like the ones of conventional needles the flow of irrigation may be restricted which is not the case with the inventive needles. If a needle having a surface as shown in figures 1 to 8 is used together with a sleeve irrigation will be increased because the space between the surface and the inner part of the sleeve is increased. However, especially the needles as shown in figures 5, 7 and 8 are atraumatic to the tissue when they are used without a sleeve.

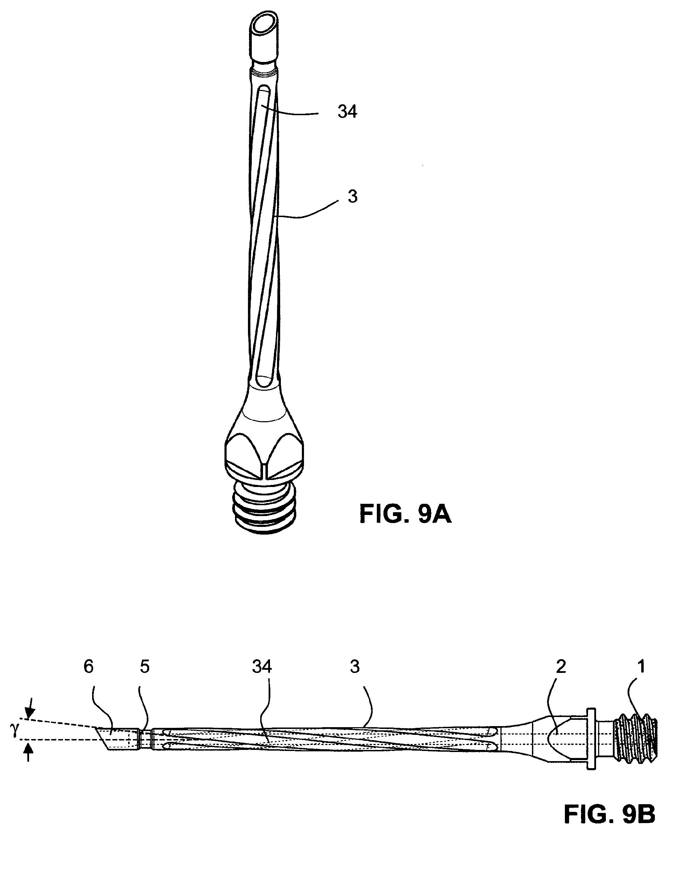

Figures 9a and 9b show a needle according to a ninth embodiment of the present invention. It has been realized in the present invention that a reflux of anterior chamber fluid and/or externally supplied irrigation fluid along the outer surface of the needle from the tip region of the needle towards the hub region may occur when the needle is used without a sleeve. This reflux should be maximized in order to achieve optimum cooling of the outer surface for avoiding corneal or scleral damage. This is achieved by providing ribs or grooves having a helical form, where the pitch angle (helix angle) is sufficiently small, but not zero. In particular, it has turned out that a pitch angle in the range of 3 to 30 degrees, preferably 5 to 15 degrees, most preferred 5 to 10 degrees, provides optimum reflux conditions. Grooves are preferred over ribs. Here, the pitch angle is defined as the angle of the ribs or grooves relative to the long axis of the shaft. In particular, Figures 9a and 9b show a needle in which helical grooves 34 are provided on the outer surface 30 of shaft 3 for which the pitch angle γ is approximately 8 degrees.

### List of reference numbers

- 1: threaded portion
- 2: hub
- 3: shaft
- 30: ribbed outer surface
- 31: grooves
- 32: ribs
- 33: depressions
- 34: helical grooves
- 4: lumen
- 40: first step
- 41: second step
- 5: tapered portion
- 6: tip
- 60: extension portion
- 61: angled portion
- 62: end portion
- 7: slit
- A: axis
- d1: first diameter
- d2: second diameter
- d3: third diameter
- D1: outside diameter of the shaft
- D2: outside diameter of the tip
- L: length of the tip
- L': length of tapered portion
- X: distance between two ribs
- Y: thickness of a rib

## Claims

1. A phacoemulsification needle comprising
a shaft with a first and a second end,
a tip, disposed at said first end of said shaft and
an aspiration lumen extending through said shaft and said tip, the tip having an opening communicating with said aspiration lumen,
wherein said shaft comprises at least one portion having protrusions on its outer surface.

2. The needle according to claim 1, wherein each protrusion has a convex shape.

3. The needle according to claim 1, wherein said protrusions are ribs having a helical form with a pitch angle between 3 and 30 degrees.

4. The needle according to claim 1, wherein said protrusions are ribs in the shape of rings.

5. The needle according to claim 4, wherein the rings define an area being perpendicular to a longitudinal axis of the shaft.

6. The needle according to claim 4, wherein the rings are arranged equidistantly along a longitudinal axis of the shaft.

7. A phacoemulsification needle comprising
a shaft with a first and a second end,
a tip, disposed at said first end of said shaft and
an aspiration lumen extending through said shaft and said tip, the tip having an opening communicating with said aspiration lumen,
wherein said shaft comprises at least one portion having depressions in its outer surface.

8. The needle according to claim 7, wherein each depression has a concave shape.

9. The needle according to claim 7, wherein said depressions are grooves having a helical form with a pitch angle between 3 and 30 degrees.

10. The needle according to claim 7, wherein said depressions are ribs in the shape of rings.

11. The needle according to claim 10, wherein the rings define an area being perpendicular to a longitudinal axis of the shaft.

12. The needle according to claim 10, wherein the rings are arranged equidistantly along a longitudinal axis of the shaft.

13. The needle according to claim 7, wherein said depressions are elongated grooves.

14. The needle according to claim 13, wherein said grooves extend approximately parallel to a longitudinal axis of said shaft.

15. The needle according to claim 13, wherein said grooves extend almost along the whole length of said shaft.

16. The needle according to claim 13, wherein said grooves are rectilinear.

17. The needle according to claim 13, wherein said grooves are arranged in an equidistant way from each other.
